# EUROPEAN PATENT SPECIFICATION

(11) **EP 3 541 211 B1**
(45) Date of publication and mention of the grant of the patent: **29.03.2023**
(21) Application number: 17808601.3
(22) Date of filing: 15.11.2017
(51) Int. Cl.: A24F 40/53, A24F 40/42

(54) **TWO-WIRE AUTHENTICATION SYSTEM FOR AN AEROSOL DELIVERY DEVICE**
ZWEIDRAHT-AUTHENTIFIZIERUNGSSYSTEM FÜR EINE AEROSOLABGABEVORRICHTUNG
SYSTÈME D'AUTHENTIFICATION À DEUX FILS POUR UN DISPOSITIF DE DISTRIBUTION D'AÉROSOL

(30) Priority: 15.11.2016 US 201615352078
(43) Date of publication of application: 25.09.2019
(60) Divisional application: 23158340.2
(73) Proprietor: RAI Strategic Holdings, Inc., Winston-Salem, NC 27101 (US)
(72) Inventor: ROGERS, James W., Cornelius NC 28031 (US); PHILLIPS, Percy, Pfafftown North Carolina 27040 (US)
(74) Representative: D Young & Co LLP
(86) International application number: PCT/IB2017/057136
(87) International publication number: WO 2018/092036

(56) References cited:
- WO-A1-2016/156609
- US-A1- 2014 096 781
- US-A1- 2014 261 495

## Description

### TECHNOLOGICAL FIELD

The present disclosure relates to aerosol delivery devices such as smoking articles, and more particularly to aerosol delivery devices that may utilize electrically generated heat for the production of aerosol (e.g., smoking articles commonly referred to as electronic cigarettes). The smoking articles may be configured to heat an aerosol precursor, which may incorporate materials that may be made or derived from, or otherwise incorporate tobacco, the precursor being capable of forming an inhalable substance for human consumption.

### BACKGROUND

Many devices have been proposed through the years as improvements upon, or alternatives to, smoking products that require combusting tobacco for use. Many of those devices purportedly have been designed to provide the sensations associated with cigarette, cigar, or pipe smoking, but without delivering considerable quantities of incomplete combustion and pyrolysis products that result from the burning of tobacco. To this end, there have been proposed numerous alternative smoking products, flavor generators, and medicinal inhalers that utilize electrical energy to vaporize or heat a volatile material, or attempt to provide the sensations of cigarette, cigar, or pipe smoking without burning tobacco to a significant degree. See, for example, the various alternative smoking articles, aerosol delivery devices and heat generating sources set forth in the background art described in U.S. Pat. No. 8,881,737 to Collett et al., U.S. Pat. App. Pub. No. 2013/0255702 to Griffith Jr. et al., U.S. Pat. App. Pub. No. 2014/0000638 to Sebastian et al., U.S. Pat. App. Pub. No. 2014/0096781 to Sears et al., U.S. Pat. App. Pub. No. 2014/0096782 to Ampolini et al., U.S. Pat. App. Pub. No. 2015/0059780 to Davis et al., and U.S. Pat. App. Ser. No. 15/222,615 to Watson et al., filed July 28, 2016. See also, for example, the various embodiments of products and heating configurations described in the background sections of U.S. Pat. Nos. 5,388,594 to Counts et al. and 8,079,371 to Robinson et al.

However, it may be desirable to provide a two-wire authentication system for authenticating and directing power within an aerosol delivery device.

US 2014/0096781 A1 discloses a smoking article, comprising a control body portion having a control body engagement end, the control body portion having a first control component therein; and a cartridge body portion including a cartridge body engagement end configured to removably engage the control body engagement end, the cartridge body portion further including a consumable arrangement comprising at least an aerosol precursor composition and at least one heating element operably engaged therewith, and a second control component, the consumable arrangement being configured to be in communication with the first control component upon engagement between the cartridge body and control body portions.

### BRIEF SUMMARY

The present disclosure relates to an aerosol delivery device and a control body of an aerosol delivery device as set out in claims 1 and 7.

### BRIEF DESCRIPTION OF THE DRAWING(S)

Having thus described the disclosure in the foregoing general terms, reference will now be made to the accompanying drawings, which are not necessarily drawn to scale, and wherein:
Figure 1 illustrates a side view of an aerosol delivery device including a cartridge coupled to a control body according to an example implementation of the present disclosure;
Figure 2 is a partially cut-away view of the aerosol delivery device according to various example implementations;
Figure 3 illustrates various elements of a control body and cartridge of the aerosol delivery device, according to various example implementations; and
Figures 4 and 5 illustrate suitable switching circuits of the control body and cartridge of Figures 1, 2 and 3, accordingly to various example implementations.

### DETAILED DESCRIPTION

The present disclosure will now be described more fully hereinafter with reference to example implementations thereof. These example implementations are described so that this disclosure will be thorough and complete, and will fully convey the scope of the disclosure to those skilled in the art. Indeed, the disclosure may be embodied in many different forms and should not be construed as limited to the implementations set forth herein; rather, these implementations are provided so that this disclosure will satisfy applicable legal requirements. As used in the specification and the appended claims, the singular forms "a," "an," "the" and the like include plural referents unless the context clearly dictates otherwise.

As described hereinafter, example implementations of the present disclosure relate to aerosol delivery systems. Aerosol delivery systems according to the present disclosure use electrical energy to heat a material (preferably without combusting the material to any significant degree) to form an inhalable substance; and components of such systems have the form of articles most preferably are sufficiently compact to be considered hand-held devices. That is, use of components of preferred aerosol delivery systems does not result in the production of smoke in the sense that aerosol results principally from byproducts of combustion or pyrolysis of tobacco, but rather, use of those preferred systems results in the production of vapors resulting from volatilization or vaporization of certain components incorporated therein. In some example implementations, components of aerosol delivery systems may be characterized as electronic cigarettes, and those electronic cigarettes most preferably incorporate tobacco and/or components derived from tobacco, and hence deliver tobacco derived components in aerosol form.

Aerosol generating pieces of certain preferred aerosol delivery systems may provide many of the sensations (e.g., inhalation and exhalation rituals, types of tastes or flavors, organoleptic effects, physical feel, use rituals, visual cues such as those provided by visible aerosol, and the like) of smoking a cigarette, cigar or pipe that is employed by lighting and burning tobacco (and hence inhaling tobacco smoke), without any substantial degree of combustion of any component thereof. For example, the user of an aerosol generating piece of the present disclosure can hold and use that piece much like a smoker employs a traditional type of smoking article, draw on one end of that piece for inhalation of aerosol produced by that piece, take or draw puffs at selected intervals of time, and the like.

Aerosol delivery systems of the present disclosure also can be characterized as being vapor-producing articles or medicament delivery articles. Thus, such articles or devices can be adapted so as to provide one or more substances (e.g., flavors and/or pharmaceutical active ingredients) in an inhalable form or state. For example, inhalable substances can be substantially in the form of a vapor (i.e., a substance that is in the gas phase at a temperature lower than its critical point). Alternatively, inhalable substances can be in the form of an aerosol (i.e., a suspension of fine solid particles or liquid droplets in a gas). For purposes of simplicity, the term "aerosol" as used herein is meant to include vapors, gases and aerosols of a form or type suitable for human inhalation, whether or not visible, and whether or not of a form that might be considered to be smoke-like.

Aerosol delivery systems of the present disclosure generally include a number of components provided within an outer body or shell, which may be referred to as a housing. The overall design of the outer body or shell can vary, and the format or configuration of the outer body that can define the overall size and shape of the aerosol delivery device can vary. Typically, an elongated body resembling the shape of a cigarette or cigar can be a formed from a single, unitary housing or the elongated housing can be formed of two or more separable bodies. For example, an aerosol delivery device can comprise an elongated shell or body that can be substantially tubular in shape and, as such, resemble the shape of a conventional cigarette or cigar. In one example, all of the components of the aerosol delivery device are contained within one housing. Alternatively, an aerosol delivery device can comprise two or more housings that are joined and are separable. For example, an aerosol delivery device can possess at one end a control body comprising a housing containing one or more reusable components (e.g., an accumulator such as a rechargeable battery, thin film solid state battery and/or capacitor, and various electronics for controlling the operation of that article), and at the other end and removably coupleable thereto, an outer body or shell containing a disposable portion (e.g., a disposable flavor-containing cartridge).

Aerosol delivery systems of the present disclosure most preferably comprise some combination of a power source (i.e., an electrical power source), at least one control component (e.g., means for actuating, controlling, regulating and ceasing power for heat generation, such as by controlling electrical current flow the power source to other components of the article - e.g., a microprocessor, individually or as part of a microcontroller), a heater or heat generation member (e.g., an electrical resistance heating element or other component, which alone or in combination with one or more further elements may be commonly referred to as an "atomizer"), an aerosol precursor composition (e.g., commonly a liquid capable of yielding an aerosol upon application of sufficient heat, such as ingredients commonly referred to as "smoke juice," "e-liquid" and "e-juice"), and a mouthend region or tip for allowing draw upon the aerosol delivery device for aerosol inhalation (e.g., a defined airflow path through the article such that aerosol generated can be withdrawn therefrom upon draw).

More specific formats, configurations and arrangements of components within the aerosol delivery device of the present disclosure will be evident in light of the further disclosure provided hereinafter. Additionally, the selection of various aerosol delivery device components can be appreciated upon consideration of the commercially available electronic aerosol delivery devices. Further, the arrangement of the components within the aerosol delivery device can also be appreciated upon consideration of the commercially available electronic aerosol delivery devices. Examples of commercially available products, for which the components thereof, methods of operation thereof, materials included therein, and/or other attributes thereof may be included in the devices of the present disclosure have been marketed as ACCORD^{®} by Philip Morris Incorporated; ALPHA^{™}, JOYE 510^{™} and M4^{™} by InnoVapor LLC; CIRRUS^{™} and FLING^{™} by White Cloud Cigarettes; BLU^{™} by Lorillard Technologies, Inc.; COHITA^{™}, COLIBRI^{™}, ELITE CLASSIC^{™}, MAGNUM^{™}, PHANTOM^{™} and SENSE^{™} by Epuffer^{®} International Inc.; DUOPRO^{™}, STORM^{™} and VAPORKING^{®} by Electronic Cigarettes, Inc.; EGAR^{™} by Egar Australia; eGo-C^{™} and eGo-T^{™} by Joyetech; ELUSION^{™} by Elusion UK Ltd; EONSMOKE^{®} by Eonsmoke LLC; FIN^{™} by FIN Branding Group, LLC; SMOKE^{®} by Green Smoke Inc. USA; GREENARETTE^{™} by Greenarette LLC; HALLIGAN^{™}, HENDU^{™}, JET^{™}, MAXXQ^{™}, PINK^{™} and PITBULL^{™} by Smoke Stik^{®}; HEATBAR^{™} by Philip Morris International, Inc.; HYDRO IMPERIAL^{™} and LXE^{™} from Crown7; LOGIC^{™} and THE CUBAN^{™} by LOGIC Technology; LUCI^{®} by Luciano Smokes Inc.; METRO^{®} by Nicotek, LLC; NJOY^{®} and ONEJOY^{™} by Sottera, Inc.; NO. 7^{™} by SS Choice LLC; PREMIUM ELECTRONIC CIGARETTE^{™} by PremiumEstore LLC; RAPP E-MYSTICK^{™} by Ruyan America, Inc.; RED DRAGON^{™} by Red Dragon Products, LLC; RUYAN^{®} by Ruyan Group (Holdings) Ltd.; SF^{®} by Smoker Friendly International, LLC; GREEN SMART SMOKER^{®} by The Smart Smoking Electronic Cigarette Company Ltd.; SMOKE ASSIST^{®} by Coastline Products LLC; SMOKING EVERYWHERE^{®} by Smoking Everywhere, Inc.; V2CIGS^{™} by VMR Products LLC; VAPOR NINE^{™} by VaporNine LLC; VAPOR4LIFE^{®} by Vapor 4 Life, Inc.; VEPPO^{™} by E-CigaretteDirect, LLC; AVIGO, VUSE, VUSE CONNECT, VUSE FOB, VUSE HYBRID, ALTO, ALTO+, MODO, CIRO, FOX + FOG, AND SOLO+ by R. J. Reynolds Vapor Company; MISTIC MENTHOL by Mistic Ecigs; and VYPE by CN Creative Ltd. Yet other electrically powered aerosol delivery devices, and in particular those devices that have been characterized as so-called electronic cigarettes, have been marketed under the tradenames COOLER VISIONS^{™}; DIRECT E-CIG^{™}; DRAGONFLY^{™}; EMIST^{™}; EVERSMOKE^{™}; GAMUCCI^{®}; HYBRID FLAME^{™}; KNIGHT STICKS^{™}; ROYAL BLUES^{™}; SMOKETIP^{®}; SOUTH BEACH SMOKE^{™}.

Additional manufacturers, designers, and/or assignees of components and related technologies that may be employed in the aerosol delivery device of the present disclosure include Shenzhen Jieshibo Technology of Shenzhen, China; Shenzhen First Union Technology of Shenzhen City, China; Safe Cig of Los Angeles, CA; Janty Asia Company of the Philippines; Joyetech Changzhou Electronics of Shenzhen, China; SIS Resources; B2B International Holdings of Dover, DE; Evolv LLC of OH; Montrade of Bologna, Italy; Shenzhen Bauway Technology of Shenzhen, China; Global Vapor Trademarks Inc. of Pompano Beach, FL; Vapor Corp. of Fort Lauderdale, FL; Nemtra GMBH of Raschau-Markersbach, Germany, Perrigo L. Co. of Allegan, MI; Needs Co., Ltd.; Smokefree Innotec of Las Vegas, NV; McNeil AB of Helsingborg, Sweden; Chong Corp; Alexza Pharmaceuticals of Mountain View, CA; BLEC, LLC of Charlotte, NC; Gaitrend Sari of Rohrbach-lès-Bitche, France; FeelLife Bioscience International of Shenzhen, China; Vishay Electronic BMGH of Selb, Germany; Shenzhen Smaco Technology Ltd. of Shenzhen, China; Vapor Systems International of Boca Raton, FL; Exonoid Medical Devices of Israel; Shenzhen Nowotech Electronic of Shenzhen, China; Minilogic Device Corporation of Hong Kong, China; Shenzhen Kontle Electronics of Shenzhen, China, and Fuma International, LLC of Medina, OH, 21st Century Smoke of Beloit, WI, and Kimree Holdings (HK) Co. Limited of Hong Kong, China.

In various examples, an aerosol delivery device can comprise a reservoir configured to retain the aerosol precursor composition. The reservoir particularly can be formed of a porous material (e.g., a fibrous material) and thus may be referred to as a porous substrate (e.g., a fibrous substrate).

A fibrous substrate useful as a reservoir in an aerosol delivery device can be a woven or nonwoven material formed of a plurality of fibers or filaments and can be formed of one or both of natural fibers and synthetic fibers. For example, a fibrous substrate may comprise a fiberglass material. In particular examples, a cellulose acetate material can be used. In other example implementations, a carbon material can be used. A reservoir may be substantially in the form of a container and may include a fibrous material included therein.

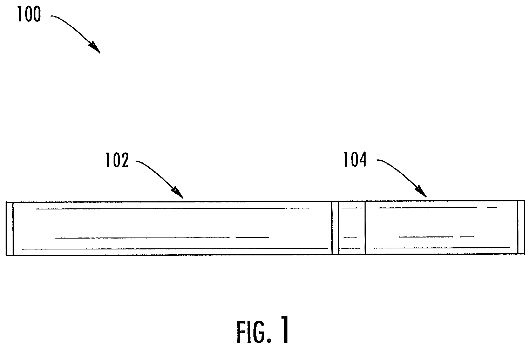

Figure 1 illustrates a side view of an aerosol delivery device **100** including a control body **102** and a cartridge **104**, according to various example implementations of the present disclosure. In particular, Figure 1 illustrates the control body and the cartridge coupled to one another. The control body and the cartridge may be detachably aligned in a functioning relationship. Various mechanisms may connect the cartridge to the control body to result in a threaded engagement, a press-fit engagement, an interference fit, a magnetic engagement or the like. The aerosol delivery device may be substantially rod-like, substantially tubular shaped, or substantially cylindrically shaped in some example implementations when the cartridge and the control body are in an assembled configuration. The aerosol delivery device may also be substantially rectangular or rhomboidal in cross-section, which may lend itself to greater compatibility with a substantially flat or thin-film power source, such as a power source including a flat battery. The cartridge and control body may include separate, respective housings or outer bodies, which may be formed of any of a number of different materials. The housing may be formed of any suitable, structurally-sound material. In some examples, the housing may be formed of a metal or alloy, such as stainless steel, aluminum or the like. Other suitable materials include various plastics (e.g., polycarbonate), metal-plating over plastic, ceramics and the like.

In some example implementations, one or both of the control body **102** or the cartridge **104** of the aerosol delivery device **100** may be referred to as being disposable or as being reusable. For example, the control body may have a replaceable battery or a rechargeable battery and thus may be combined with any type of recharging technology, including connection to a typical alternating current electrical outlet, connection to a car charger (i.e., a cigarette lighter receptacle), connection to a computer, such as through a universal serial bus (USB) cable or connector, or connection to a photovoltaic cell (sometimes referred to as a solar cell) or solar panel of solar cells. Further, in some example implementations, the cartridge may comprise a single-use cartridge, as disclosed in U.S. Pat. No. 8,910,639 to Chang et al.

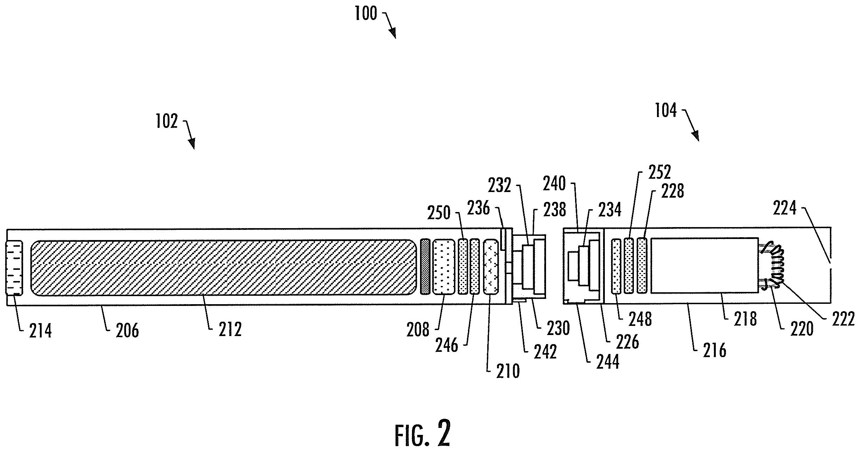

Figure 2 more particularly illustrates the aerosol delivery device 100, in accordance with some example implementations. As seen in the cut-away view illustrated therein, again, the aerosol delivery device can comprise a control body 102 and a cartridge 104 each of which include a number of respective components. The components illustrated in Figure 2 are representative of the components that may be present in a control body and cartridge and are not intended to limit the scope of components that are encompassed by the present disclosure. As shown, for example, the control body can be formed of a control body shell **206** that can include one or more of each of a number of electronic components, such as a control component **208** (e.g., a microprocessor, individually or as part of a microcontroller), a flow sensor **210**, a power source **212** and/or light-emitting diode (LED) **214**, and such components can be variably aligned. The power source may include, for example, a battery (single-use or rechargeable), solid-state battery, thin-film solid-state battery, supercapacitor or the like, or some combination thereof. Some examples of a suitable power source are provided in U.S. Pat. App. Ser. No. 14/918,926 to Sur et al., filed October 21, 2015. The LED may be one example of a suitable visual indicator with which the aerosol delivery device **100** may be equipped. Other indicators such as audio indicators (e.g., speakers), haptic indicators (e.g., vibration motors) or the like can be included in addition to or as an alternative to visual indicators such as the LED.

The cartridge **104** can be formed of a cartridge shell **216** enclosing a reservoir **218** configured to retain the aerosol precursor composition, and including a heater **220** (sometimes referred to as a heating element). As shown, in some examples, the reservoir may be in fluid communication with a liquid transport element **222** adapted to wick or otherwise transport an aerosol precursor composition stored in the reservoir housing to the heater. In some example, a valve may be positioned between the reservoir and heater, and configured to control an amount of aerosol precursor composition passed or delivered from the reservoir to the heater. In various configurations, the structure including at least the shell, reservoir and heater may be referred to as a tank; and accordingly, the terms "cartridge," "tank" and the like may be used interchangeably to refer to a shell or other housing enclosing a reservoir for aerosol precursor composition, and including a heater.

Various examples of materials configured to produce heat when electrical current is applied therethrough may be employed to form the heater **220**. The heater in these examples may be a resistive heating element such as a wire coil, micro heater or the like. Example materials from which the wire coil may be formed include Kanthal (FeCrAl), Nichrome, Molybdenum disilicide (MoSi₂), molybdenum silicide (MoSi), Molybdenum disilicide doped with Aluminum (Mo(Si,Al)₂), graphite and graphite-based materials (e.g., carbon-based foams and yarns) and ceramics (e.g., positive or negative temperature coefficient ceramics). Example implementations of heaters or heating members useful in aerosol delivery devices according to the present disclosure are further described below, and can be incorporated into devices such as illustrated in Figure 2 as described herein.

An opening **224** may be present in the cartridge shell **216** (e.g., at the mouthend) to allow for egress of formed aerosol from the cartridge **104**.

The cartridge **104** also may include one or more electronic components, which may include an integrated circuit, a memory component, a sensor, or the like. The electronic components may be adapted to communicate with the control component **208** and/or with an external device by wired or wireless means. The electronic components may be positioned anywhere within the cartridge or a base **226** thereof.

As explained in greater detail below, for example, the electronic components of the cartridge **104** may include an authentication device **228** to deter or prevent counterfeit cartridges from being used with the control body **102**. Examples of suitable authentication devices include the bq26150 authentication device from Texas Instruments, the ATSHA204 and ATSHA204A authentication devices from Atmel Corporation, and the like. Although not separately shown, an additional memory unit associated with the authentication device may be used to store a depletion amount of the cartridge unit, as well as to store other programmable features and information associated with the cartridge unit.

The control component **208** may be configured to communicate with the authentication device **228** to authenticate the cartridge **104** for use with the control body **102**. This authentication may be initiated and carried out in a number of different manners. In some examples, the control component may be configured to communicate with the authentication device at the initiation of every puff on the device **100** to validate the cartridge as being a legitimate device for use with the control body. An error condition may result in instances in which the cartridge is not authorized, and this error condition may be indicated by one or more visual, audio or haptic indicators. Otherwise, the control component may permit the puff to continue in instances in which the cartridge is authorized, which may include the control component causing the heater **220** to activate and vaporize aerosol precursor composition. More information regarding authentication according to aspects of the present disclosure may be found in U.S. Pat. App. Pub. No. 2014/0270727 to Ampolini et al.

Although electronic components such as the control component **208** and flow sensor **210** are illustrated separately, it is understood that various electronic components may be combined on an electronic printed circuit board (PCB) that supports and electrically connects the electronic components. Further, the PCB may be positioned horizontally relative the illustration of Figure 1 in that the PCB can be lengthwise parallel to the central axis of the control body. In some examples, one or more electronic components may comprise their own respective PCBs or other base elements to which they can be attached. In some examples, a flexible PCB may be utilized. A flexible PCB may be configured into a variety of shapes, include substantially tubular shapes. In some examples, a flexible PCB may be combined with, layered onto, or form part or all of a heater substrate.

The control body **102** and the cartridge **104** may include components adapted to facilitate a fluid engagement therebetween. As illustrated in Figure 2, the control body can include a coupler **230** having a cavity **232** therein. The base **226** of the cartridge can be adapted to engage the coupler and can include a projection **234** adapted to fit within the cavity. Such engagement can facilitate a stable connection between the control body and the cartridge as well as establish an electrical connection between the power source **212** and control component **208** in the control body and the heater **220** in the cartridge. Further, the control body shell **206** can include an air intake **236**, which may be a notch in the shell where it connects to the coupler that allows for passage of ambient air around the coupler and into the shell where it then passes through the cavity of the coupler and into the cartridge through the projection.

A coupler and a base useful according to the present disclosure are described in U.S. Pat. App. Pub. No. 2014/0261495 to Novak et al. For example, the coupler **230** as seen in Figure 2 may define an outer periphery **238** configured to mate with an inner periphery **240** of the base **226**. In one example the inner periphery of the base may define a radius that is substantially equal to, or slightly greater than, a radius of the outer periphery of the coupler. Further, the coupler may define one or more protrusions **242** at the outer periphery configured to engage one or more recesses **244** defined at the inner periphery of the base. However, various other examples of structures, shapes and components may be employed to couple the base to the coupler. In some examples the connection between the base of the cartridge **104** and the coupler of the control body **102** may be substantially permanent, whereas in other examples the connection therebetween may be releasable such that, for example, the control body may be reused with one or more additional cartridges that may be disposable and/or refillable.

The aerosol delivery device **100** may be substantially rod-like or substantially tubular shaped or substantially cylindrically shaped in some examples. In other examples, further shapes and dimensions are encompassed - e.g., a rectangular or triangular cross-section, multifaceted shapes, or the like.

The reservoir **218** illustrated in Figure 2 can be a container or can be a fibrous reservoir, as presently described. For example, the reservoir can comprise one or more layers of nonwoven fibers substantially formed into the shape of a tube encircling the interior of the cartridge shell **216**, in this example. An aerosol precursor composition can be retained in the reservoir. Liquid components, for example, can be sorptively retained by the reservoir. The reservoir can be in fluid connection with the liquid transport element **222**. The liquid transport element can transport the aerosol precursor composition stored in the reservoir via capillary action to the heater **220** that is in the form of a metal wire coil in this example. As such, the heater is in a heating arrangement with the liquid transport element. Example implementations of reservoirs and transport elements useful in aerosol delivery devices according to the present disclosure are further described below, and such reservoirs and/or transport elements can be incorporated into devices such as illustrated in Figure 2 as described herein. In particular, specific combinations of heating members and transport elements as further described below may be incorporated into devices such as illustrated in Figure 2 as described herein.

In use, when a user draws on the aerosol delivery device **100**, airflow is detected by the flow sensor **210**, and the heater **220** is activated to vaporize components of the aerosol precursor composition. Drawing upon the mouthend of the aerosol delivery device causes ambient air to enter the air intake **236** and pass through the cavity **232** in the coupler **230** and the central opening in the projection **234** of the base **226**. In the cartridge **104**, the drawn air combines with the formed vapor to form an aerosol. The aerosol is whisked, aspirated or otherwise drawn away from the heater and out the opening **224** in the mouthend of the aerosol delivery device.

In some examples, the aerosol delivery device **100** may include a number of additional software-controlled functions. For example, the aerosol delivery device may include a power-source protection circuit configured to detect power-source input, loads on the power-source terminals, and charging input. The power-source protection circuit may include short-circuit protection and under-voltage lock out. The aerosol delivery device may also include components for ambient temperature measurement, and its control component **208** may be configured to control at least one functional element to inhibit power-source charging - particularly of any battery - if the ambient temperature is below a certain temperature (e.g., 0 °C) or above a certain temperature (e.g., 45 °C) prior to start of charging or during charging.

Power delivery from the power source **212** may vary over the course of each puff on the device **100** according to a power control mechanism. The device may include a "long puff' safety timer such that in the event that a user or component failure (e.g., flow sensor **210**) causes the device to attempt to puff continuously, the control component **208** may control at least one functional element to terminate the puff automatically after some period of time (e.g., four seconds). Further, the time between puffs on the device may be restricted to less than a period of time (e.g., 100 seconds). A watchdog safety timer may automatically reset the aerosol delivery device if its control component or software running on it becomes unstable and does not service the timer within an appropriate time interval (e.g., eight seconds). Further safety protection may be provided in the event of a defective or otherwise failed flow sensor, such as by permanently disabling the aerosol delivery device in order to prevent inadvertent heating. A puffing limit switch may deactivate the device in the event of a pressure sensor fail causing the device to continuously activate without stopping after the four second maximum puff time.

The aerosol delivery device **100** may include a puff tracking algorithm configured for heater lockout once a defined number of puffs has been achieved for an attached cartridge (based on the number of available puffs calculated in light of the e-liquid charge in the cartridge). The aerosol delivery device may include a sleep, standby or low-power mode function whereby power delivery may be automatically cut off after a defined period of non-use. Further safety protection may be provided in that all charge/discharge cycles of the power source **212** may be monitored by the control component **208** over its lifetime. After the power source has attained the equivalent of a predetermined number (e.g., 200) of full discharge and full recharge cycles, it may be declared depleted, and the control component may control at least one functional element to prevent further charging of the power source.

The various components of an aerosol delivery device according to the present disclosure can be chosen from components described in the art and commercially available. Examples of batteries that can be used according to the disclosure are described in U.S. Pat. App. Pub. No. 2010/0028766 to Peckerar et al.

The aerosol delivery device **100** can incorporate the flow sensor **210** or another sensor or detector for control of supply of electric power to the heater **220** when aerosol generation is desired (e.g., upon draw during use). As such, for example, there is provided a manner or method of turning off power to the heater when the aerosol delivery device is not be drawn upon during use, and for turning on power to actuate or trigger the generation of heat by the heater during draw. Additional representative types of sensing or detection mechanisms, structure and configuration thereof, components thereof, and general methods of operation thereof, are described in U.S. Pat. No. 5,261,424 to Sprinkel, Jr., U.S. Pat. No. 5,372,148 to McCafferty et al., and PCT Pat. App. Pub. No. WO 2010/003480 to Flick.

The aerosol delivery device **100** most preferably incorporates the control component **208** or another control mechanism for controlling the amount of electric power to the heater **222** during draw. Representative types of electronic components, structure and configuration thereof, features thereof, and general methods of operation thereof, are described in U.S. Pat. No. 4,735,217 to Gerth et al., U.S. Pat. No. 4,947,874 to Brooks et al., U.S. Pat. No. 5,372,148 to McCafferty et al., U.S. Pat. No. 6,040,560 to Fleischhauer et al., U.S. Pat. No. 7,040,314 to Nguyen et al., U.S. Pat. No. 8,205,622 to Pan, U.S. Pat. App. Pub. No. 2009/0230117 to Fernando et al., U.S. Pat. App. Pub. No. 2014/0060554 to Collet et al., U.S. Pat. App. Pub. No. 2014/0270727 to Ampolini et al., and U.S. Pat. App. Pub. No. 2015/0257445 to Henry et al.

Representative types of substrates, reservoirs or other components for supporting the aerosol precursor are described in U.S. Pat. No. 8,528,569 to Newton, U.S. Pat. App. Pub. No. 2014/0261487 to Chapman et al., U.S. Pat. App. Pub. No. 2015/0059780 to Davis et al., and U.S. Pat. App. Pub. No. 2015/0216232 to Bless et al. Additionally, various wicking materials, and the configuration and operation of those wicking materials within certain types of electronic cigarettes, are set forth in U.S. Pat. App. Pub. No. 2014/0209105 to Sears et al.

The aerosol precursor composition, also referred to as a vapor precursor composition, may comprise a variety of components including, by way of example, a polyhydric alcohol (e.g., glycerin, propylene glycol or a mixture thereof), nicotine, tobacco, tobacco extract and/or flavorants. Representative types of aerosol precursor components and formulations also are set forth and characterized in U.S. Pat. No. 7,217,320 to Robinson et al. and U.S. Pat. Pub. Nos. 2013/0008457 to Zheng et al.; 2013/0213417 to Chong et al.; 2014/0060554 to Collett et al.; 2015/0020823 to Lipowicz et al.; and 2015/0020830 to Koller, as well as WO 2014/182736 to Bowen et al., and U.S. Pat. App. Ser. No. 15/222,615 to Watson et al., filed July 28, 2016. Other aerosol precursors that may be employed include the aerosol precursors that have been incorporated in the VUSE^{®} product by R. J. Reynolds Vapor Company, the BLU^{™} product by Imperial Tobacco Group PLC, the MISTIC MENTHOL product by Mistic Ecigs, and the VYPE product by CN Creative Ltd. Also desirable are the so-called "smoke juices" for electronic cigarettes that have been available from Johnson Creek Enterprises LLC.

Additional representative types of components that yield visual cues or indicators may be employed in the aerosol delivery device **100**, such as visual indicators and related components, audio indicators, haptic indicators and the like. Examples of suitable LED components, and the configurations and uses thereof, are described in U.S. Pat. No. 5,154,192 to Sprinkel et al., U.S. Pat. No. 8,499,766 to Newton, U.S. Pat. No. 8,539,959 to Scatterday, and U.S. Pat. App. Pub. No. 2015/0216233 to Sears et al.

Yet other features, controls or components that can be incorporated into aerosol delivery devices of the present disclosure are described in U.S. Pat. No. 5,967,148 to Harris et al., U.S. Pat. No. 5,934,289 to Watkins et al., U.S. Pat. No. 5,954,979 to Counts et al., U.S. Pat. No. 6,040,560 to Fleischhauer et al., U.S. Pat. No. 8,365,742 to Hon, U.S. Pat. No. 8,402,976 to Fernando et al., U.S. Pat. App. Pub. No. 2005/0016550 to Katase, U.S. Pat. App. Pub. No. 2010/0163063 to Fernando et al., U.S. Pat. App. Pub. No. 2013/0192623 to Tucker et al., U.S. Pat. App. Pub. No. 2013/0298905 to Leven et al., U.S. Pat. App. Pub. No. 2013/0180553 to Kim et al., U.S. Pat. App. Pub. No. 2014/0000638 to Sebastian et al., U.S. Pat. App. Pub. No. 2014/0261495 to Novak et al., and U.S. Pat. App. Pub. No. 2014/0261408 to DePiano et al..

As indicated above, the control component **208** includes a number of electronic components, and in some examples may be formed of a PCB. The electronic components may include a microprocessor or processor core, and a memory. In some examples, the control component may include a microcontroller with integrated processor core and memory, and which may further include one or more integrated input/output peripherals. In some examples, the control component may be coupled to a communication interface to enable wireless communication with one or more networks, computing devices or other appropriately-enabled devices. Examples of suitable communication interfaces are disclosed in U.S. Pat. App. Ser. No. 14/638,562, filed March 4, 2015, to Marion et al., the content of which is incorporated herein by reference. And examples of suitable manners according to which the aerosol delivery device may be configured to wirelessly communicate are disclosed in U.S. Pat. App. Pub. No. 2016/0007651 to Ampolini et al., and U.S. Pat. App. Pub. No. 2016/0219933 to Henry, Jr. et al..

The control component **208** may be configured to control one or more functional elements of the aerosol delivery device **100** in different states of the device. In examples in which the aerosol delivery device has a housing formed of separable bodies, the aerosol delivery device, and more particularly the control component **102**, may be in the standby mode when the control component is uncoupled with the cartridge **104**. In examples of either a unitary or separable housing, the aerosol delivery device may be in the standby mode between puffs when the control component is coupled with the cartridge. Similarly, in examples of either a unitary or separable housing, when the user draws on the device and the flow sensor **210** detects airflow, the aerosol delivery device may be placed in the active mode during which the control component may direct power from the power source **212** to power the heater **220** (heating element) and thereby control the heater to activate and vaporize components of the aerosol precursor composition.

As previously indicated, in some implementations, the control component **208** may be configured to communicate with the authentication device **228** to authenticate the cartridge **104** for use with the control body **102**. In particular, the control component may be configured to exchange authentication signals with the authentication device to authenticate the cartridge for use with the control body and, only in instances in which the cartridge is authenticated, direct power to the heating element **220** to activate and vaporize components of the aerosol precursor composition. The control component may be configured to direct power to the heating element in response to a flow of air through at least a portion of the aerosol delivery device **100**. In these implementations, the control body and cartridge may be coupled to one another and configured to exchange data (e.g., authentication data) and power therebetween using a two-wire authentication system. This configuration provides flexibility to use the control body or cartridge with other generic cartridges or control bodies, respectively, that have similar two-wire authentication systems.

As shown in Figure 2, the control body **102** may include a two-wire electrical connector **246**, and the cartridge **104** may include a corresponding two-wire electrical **248**. The two-wire electrical connectors are coupled when the control body is coupled with the cartridge. As such, the authentication signals are exchanged, and the power is directed, across the coupled two-wire electrical connectors. Further, the control body may include a first switching circuit **250**, and the cartridge may include a second switching circuit **252**. Similarly, the first and second switching circuits are coupled when the control body is coupled with the cartridge. The first and second switching circuits may be coupled to form switching circuitry configured to manage exchange of the authentication signals and direction of the power across the two-wire electrical connectors.

In some examples, the authentication signals and the power are formatted as pulse width modulation (PWM) signals that have a first frequency and a second frequency, respectively. In these examples, the first frequency is at least two times larger than the second frequency. To manage the authentication signals and the power across the two-wire electrical connector, the switching circuitry (including first and second switching circuits **250**, **252**) is configured to switch a PWM signal having the first frequency across the two-wire electrical connectors **246**, **248** between pulses of a PWM signal having the second frequency. In some examples, the first switching circuit may be or include a high-side switch operatively coupled to a bus transceiver in which the high-side switch is configured to receive and switch the PWM signal across the two-wire electrical connectors.

In some implementations, the authentication signals exchanged across the two-wire electrical connectors **246**, **248** have a voltage level at or below a predetermined threshold voltage, and the power across the two-wire electrical connectors has a voltage level above the predetermined threshold voltage. In one implementation, the predetermined threshold voltage is 2.5 volts. For example, in an instance in which a signal has a voltage level above the predetermined threshold voltage, the switching circuitry (including first and second switching circuits **250**, **252**) is configured to receive and forward the signal to the heating element **220**, as power directed thereto. In some examples, the predetermined threshold voltage corresponds to a nominal voltage of the power source **212**.

Alternatively, in an instance in which a signal has a voltage level at or below the predetermined threshold voltage, the switching circuitry (including first and second switching circuits **250**, **252**) is configured to receive and forward the signal to the authentication device **228** as one of the authentication signals. In some examples, the switching circuitry is configured to receive a plurality of signals and forward the signals of the plurality of signals to the authentication device as authentication signals. In these examples implementations, the plurality of signals are forwarded until a signal of the plurality of signals has a voltage level above the predetermined threshold voltage.

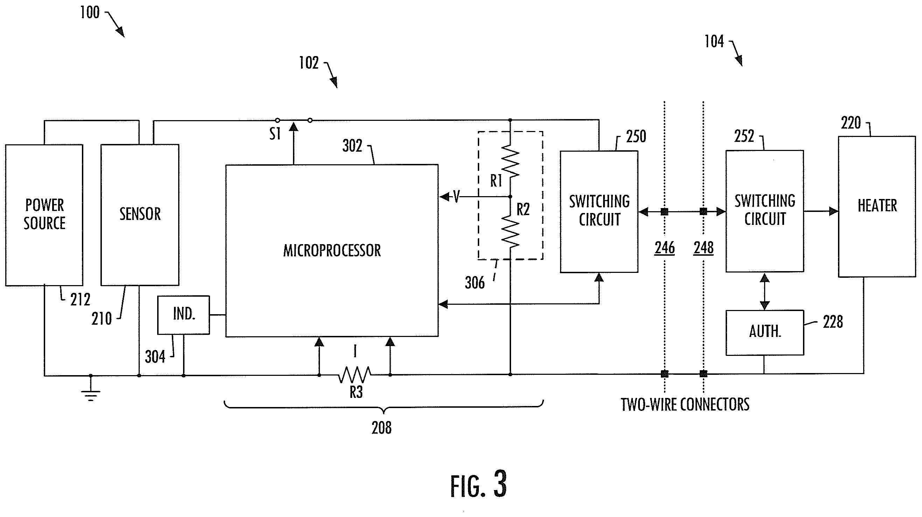

Figure 3 more particularly illustrates various interconnected electronic components of the control body **102** and cartridge **104**, according to various example implementations. As shown, the control component **208** may include a microprocessor **302** and a number of other electrical components, such as resistors, capacitors, switches and the like, which may be coupled together and with the power source **212** and heater **220** via conductors such as wires, traces or the like to form an electrical circuit. In some examples, the heater may include a communication terminal for communicating data such as the puff count.

In accordance with example implementations of the present disclosure, the microprocessor **302** may be configured to perform a number of control operations. In the active mode, for example, the microprocessor may be configured to direct power from the power source **212** (e.g., directly or through the flow sensor **210**) to turn the heater **222** on and thereby control the heater to activate and vaporize components of the aerosol precursor composition. This may include, for example, a switch **S1** between the power source and the heater, which the microprocessor may operate in a closed state, as shown in Figure 3. In some examples, the microprocessor may also control operation of at least one other functional element. One example of a suitable functional element may be an indicator **304** such as a visual, audio or haptic indicator.

In some examples, power delivery from the power source **212** may vary according to a power control mechanism, which may include the microprocessor **302** being configured to measure the voltage at a positive terminal of the heater **220** and control power to the heater based thereon. The voltage at the positive terminal may correspond to a positive heater voltage. The microprocessor may operate on the actual voltage, or an analog-to-digital converter (ADC) may be included to convert the actual voltage to a digital equivalent. In some examples, the control component **208** may include a voltage divider **306** with resistors **R1** and **R2**, which may be configured to reduce the voltage to the microprocessor.

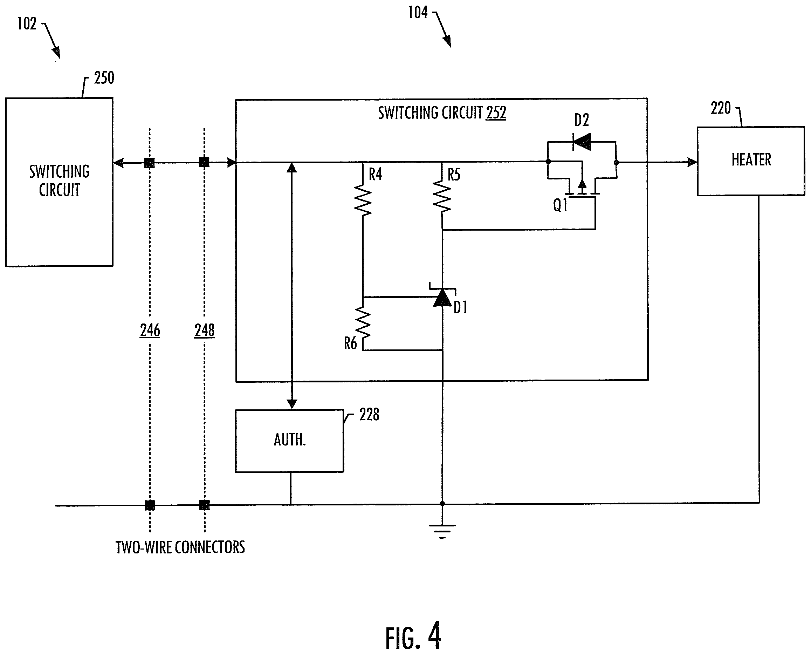

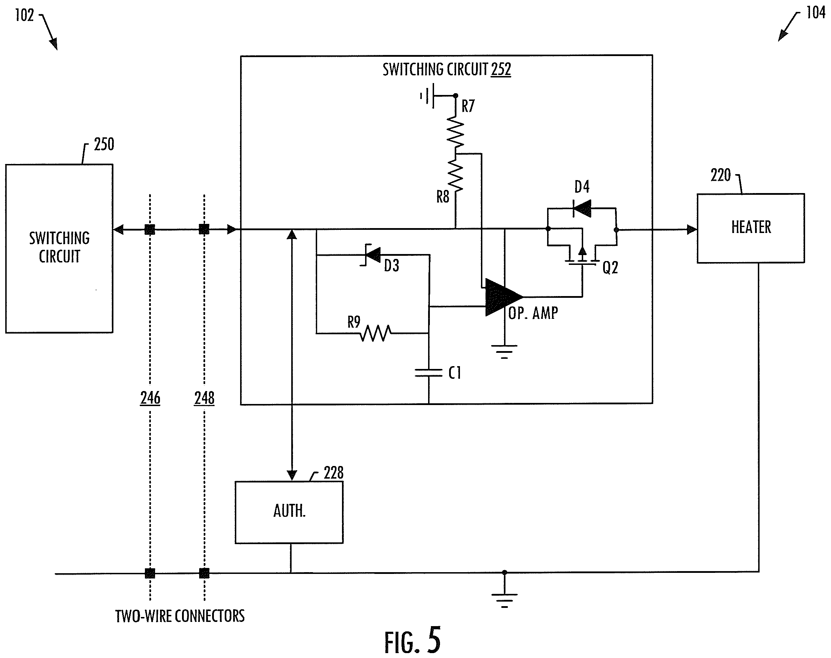

Figures 4 and 5 more particularly illustrate suitable examples of the switching circuitry (including first and second switching circuits **250**, **252**). As shown, the second switching circuit may include a plurality of electronic components (e.g., resistors, diodes, capacitors, operational amplifiers, transistors and the like). In one example, as shown in Figure 4, the second switching circuit may include a configuration of resistors **R4**, **R5** and **R6**, diodes **D1** and **D2** (e.g., traditional diodes, or a zener diodes configured to implement a voltage shunt regulator), and a transistor **Q1** (e.g., a metal-oxide-semiconductor field-effect transistor (MOSFET)) configured to receive and forward a signal to the heating element **220**, as power directed thereto in an instance in which the signal has a voltage level above the predetermined threshold voltage, or receive and forward a signal to the authentication device **228** as one of the authentication signals in an instance in which the signal has a voltage level at or below the predetermined threshold voltage.

In another example, as shown in Figure 5, the second switching circuit **252** may include a configuration of resistors **R7**, **R8** and **R9**, capacitors **C1**, diodes **D3** and **D4** (e.g., a traditional diode or schottky diode), and a transistor **Q1** (e.g., a MOSFET) configured to switch a PWM signal having a first frequency across the two-wire electrical connectors **246**, **248** between pulses of a PWM signal having a second frequency where the first frequency is at least two times larger than the second frequency. It should be noted that although the implementation of Figures 4 and 5 are illustrated separately, the second switching circuit may include both configurations of the electronic components therein.

The foregoing description of use of the article(s) can be applied to the various example implementations described herein through minor modifications, which can be apparent to the person of skill in the art in light of the further disclosure provided herein. The above description of use, however, is not intended to limit the use of the article but is provided to comply with all necessary requirements of disclosure of the present disclosure. Any of the elements shown in the article(s) illustrated in FIGS. 1-7 or as otherwise described above may be included in an aerosol delivery device according to the present disclosure.

## Claims

1. An aerosol delivery device comprising:
a cartridge (104) that is equipped with a heating element (220) and an authentication device (228), and contains an aerosol precursor composition; and
a control body (102) coupled with the cartridge (104) and configured to exchange authentication signals with the authentication device (228) to authenticate the cartridge (104) for use with the control body (102), and only in instances in which the cartridge (104) is authenticated, direct power to the heating element (220) to activate and vaporize components of the aerosol precursor composition,
wherein the control body (102) and the cartridge (104) include respectively a two-wire electrical connector (246) and a corresponding two-wire electrical connector (248) coupled with one another, and across which the authentication signals are exchanged and the power is directed, and
wherein the control body (102) and the cartridge (104) further include respectively a first switching circuit (250) and a second switching circuit (252), the first switching circuit (250) being coupled with the second switching circuit (252) to form switching circuitry configured to manage the authentication signals and the power across the two-wire electrical connector (246).

2. The aerosol delivery device of Claim 1, wherein the authentication signals across the two-wire electrical connector (246) have a voltage level at or below a predetermined threshold voltage, and the power across the two-wire electrical connector (246) has a voltage level above the predetermined threshold voltage, in particular,
wherein the predetermined threshold voltage is 2.5 volts.

3. The aerosol delivery device of Claim 2, wherein the switching circuitry being configured to manage the authentication signals and the power across the two-wire electrical connector (246) includes being configured to receive and forward a signal to the authentication device (228) as one of the authentication signals in an instance in which the signal has a voltage level at or below the predetermined threshold voltage, in particular,
wherein the switching circuitry being configured to receive and forward the signal includes being configured to receive a plurality of signals and forward signals of the plurality of signals to the authentication device (228) as authentication signals until a signal of the plurality of signals has a voltage level above the predetermined threshold voltage.

4. The aerosol delivery device of Claim 2 or 3, wherein the switching circuitry being configured to manage the authentication signals and the power across the two-wire electrical connector (246) includes being configured to receive and forward a signal to the heating element (220) as power directed thereto in an instance in which the signal has a voltage level above the predetermined threshold voltage.

5. The aerosol delivery device of any one of Claims 1 to 4, wherein the authentication signals and the power are formatted as pulse width modulation, PWM,
signals having respectively a first frequency and a second frequency, the first frequency being at least two times larger than the second frequency, in particular,
wherein the switching circuitry being configured to manage the authentication signals and the power across the two-wire electrical connector (246) includes the switching circuitry being configured to switch a PWM signal having the first frequency across the two-wire electrical connector (246) between pulses of a PWM signal having the second frequency.

6. The aerosol delivery device of any one of Claims 1 to 5, wherein the control component (208) being configured to direct power to the heating element (220) includes being configured to direct power to the heating element (220) in response to a flow of air through at least a portion of the aerosol delivery device, the air being combinable with vapor formed by vaporization of components of the aerosol precursor composition to form an aerosol.

7. A control body coupleable with a cartridge (104) that is equipped with a heating element (220) and an authentication device (228), and contains an aerosol precursor composition, the control body (102) being coupleable with the cartridge (104) to form an aerosol delivery device, the control body (102) comprising:
a two-wire electrical connector (246) coupleable with the cartridge (104) and configured to carry authentication signals and power thereacross;
a control component (208) having the two-wire electrical connector (246) coupled thereto, the control component (208) being configured to exchange the authentication signals and to direct the power across the two-wire electrical connector (246), the control component (208) processing the authentication signals from the two-wire connector (246) for authenticating the cartridge (104) and directing the power to the two-wire electrical connector (246) upon the authentication; and
a first switching circuit (250) coupled with the control component (208) and forming part of switching circuitry configured to manage the authentication signals and the power across the two-wire electrical connector (246).

8. The control body of Claim 7, wherein the authentication signals across the two-wire electrical connector (246) have a voltage level at or below a predetermined threshold voltage, and the power across the two-wire electrical connector (246) has a voltage level above the predetermined threshold voltage.

9. The control body of Claim 8, wherein the predetermined threshold voltage is 2.5 volts.

10. The control body of Claim 7 or 8, wherein the switching circuitry being configured to manage the authentication signals and the power across the two-wire electrical connector (246) includes being configured to receive and forward a signal across the two-wire electrical connector (246) as one of the authentication signals in an instance in which the signal has a voltage level at or below the predetermined threshold voltage.

11. The control body of Claim 10, wherein the switching circuitry being configured to receive and forward the signal includes being configured to receive a plurality of signals and forward signals of the plurality of signals across the two-wire electrical connector (246) as authentication signals until a signal of the plurality of signals has a voltage level above the predetermined threshold voltage.

12. The control body of any one of Claims 8 to 11, wherein the switching circuitry being configured to manage the authentication signals and the power across the two-wire electrical connector (246) includes being configured to receive and forward a signal to the heating element (220) as power directed thereto in an instance in which the signal has a voltage level above the predetermined threshold voltage.

13. The control body of any one of Claims 7 to 12, wherein the authentication signals and the power are formatted as pulse width modulation , PWM, signals having respectively a first frequency and a second frequency, the first frequency being at least two times larger than the second frequency.

14. The control body of Claim 13, wherein the switching circuitry being configured to manage the authentication signals and the power across the two-wire electrical connector (246) includes the switching circuitry being configured to switch a PWM signal having the first frequency across the two-wire electrical connector (246) between pulses of a PWM signal having the second frequency.

15. The control body of any one of Claims 7 to 14, wherein the control component (208) being configured to direct power to the heating element includes being configured to direct power to the heating element (220) in response to a flow of air through at least a portion of the aerosol delivery device, the air being combinable with vapor formed by vaporization of components of the aerosol precursor composition to form an aerosol.

## Patentansprüche

1. Aerosolabgabevorrichtung, umfassend:
eine Kartusche (104), die mit einem Heizelement (220) und einer Authentifizierungsvorrichtung (228) ausgerüstet ist und eine Aerosolvorläuferzusammensetzung enthält; und
einen Steuerkörper (102), der mit der Kartusche (104) gekoppelt ist und ausgestaltet ist, um Authentifizierungssignale mit der Authentifizierungsvorrichtung (228) auszutauschen, um die Kartusche (104) zur Verwendung mit dem Steuerkörper (102) zu authentifizieren, und um nur in Fällen, in denen die Kartusche (104) authentifiziert ist, Leistung zu dem Heizelement (220) zu lenken, um Komponenten der Aerosolvorläuferzusammensetzung zu aktivieren und zu verdampfen,
wobei der Steuerkörper (102) und die Kartusche (104) jeweils einen elektrischen Zweidrahtverbinder (246) beziehungsweise einen entsprechenden elektrischen Zweidrahtverbinder (248) einschließen, die miteinander gekoppelt sind, und worüber die Authentifizierungssignale ausgetauscht und die Leistung gelenkt wird, und
wobei der Steuerkörper (102) und die Kartusche (104) des Weiteren eine erste Schaltschaltung (250) beziehungsweise eine zweite Schaltschaltung (252) einschließen, wobei die erste Schaltschaltung (250) mit der zweiten Schaltschaltung (252) gekoppelt ist, um Schaltschaltkreis(e) zu bilden, der/die ausgestaltet ist/sind zum Management der Authentifizierungssignale und der Leistung über den elektrischen Zweidrahtverbinder (246).

2. Aerosolabgabevorrichtung nach Anspruch 1, wobei die Authentifizierungssignale über den elektrischen Zweidrahtverbinder (246) einen Spannungspegel auf oder unter einer vorbestimmten Schwellenwertspannung haben, und die Leistung über den elektrischen Zweidrahtverbinder (246) einen Spannungspegel über der vorbestimmten Schwellenwertspannung hat, wobei die vorbestimmte Schwellenwertspannung insbesondere 2,5 Volt beträgt.

3. Aerosolabgabevorrichtung nach Anspruch 2, wobei die Schaltschaltkreis(e), der/die ausgestaltet ist/sind zum Management der Authentifizierungssignale und der Leistung über den elektrischen Zweidrahtverbinder (246), einschließt/einschließen, dass er/sie ausgestaltet ist/sind zum Empfangen und Weiterleiten eines Signals an die Authentifizierungsvorrichtung (228) als eines der Authentifizierungssignale in einem Moment, in dem das Signal einen Spannungspegel auf oder unter der vorbestimmten Schwellenwertspannung hat, wobei insbesondere
der/die Schaltschaltkreis(e), der/die ausgestaltet ist/sind zum Empfangen und Weiterleiten des Signals, einschließt/einschließen, dass er/sie ausgestaltet ist/sind zum Empfangen einer Vielzahl von Signalen und zum Weiterleiten von Signalen der Vielzahl von Signalen an die Authentifizierungsvorrichtung (228) als Authentifizierungssignale, bis ein Signal von der Vielzahl der Signale einen Spannungspegel über der vorbestimmten Schwellenwertspannung hat.

4. Aerosolabgabevorrichtung nach Anspruch 2 oder 3, wobei die Schaltschaltkreis(e), der/die ausgestaltet ist/sind zum Management der Authentifizierungssignale und der Leistung über den elektrischen Zweidrahtverbinder (246), einschließt/einschließen, dass er/sie ausgestaltet ist/sind zum Empfangen und Weiterleiten eines Signals an das Heizelement (220) als Leistung, die in einem Moment, in dem das Signal einen Spannungspegel über der vorbestimmten Schwellenwertspannung hat, dorthin gelenkt wird.

5. Aerosolabgabevorrichtung nach einem der Ansprüche 1 bis 4, wobei die Authentifizierungssignale und die Leistung als Pulsbreitenmodulations, PWM, Signale mit jeweils einer ersten Frequenz und einer zweiten Frequenz formatiert sind, wobei die erste Frequenz mindestens zwei Mal größer als die zweite Frequenz ist, wobei insbesondere
der/die Schaltschaltkreis(e), der/die ausgestaltet ist/sind zum Management der Authentifizierungssignale und der Leistung über den elektrischen Zweidrahtverbinder (246), einschließt/einschließen, dass der/die Schaltschaltkreis(e) ausgestaltet ist/sind zum Schalten eines PWM-Signals mit der ersten Frequenz über den elektrischen Zweidrahtverbinder (246) zwischen Pulsen des PWM-Signals mit der zweiten Frequenz.

6. Aerosolabgabevorrichtung nach einem der Ansprüche 1 bis 5, wobei die Steuerkomponente (208), die ausgestaltet ist, um Leistung zu dem Heizelement (220) zu lenken, einschließt, dass sie ausgestaltet ist, um in Reaktion auf eine Luftströmung durch mindestens einen Anteil der Aerosolabgabevorrichtung Leistung zu dem Heizelement (220) zu lenken, wobei die Luft mit Dampf, der durch Verdampfung von Komponenten der Aerosolvorläuferzusammensetzung gebildet wird, unter Bildung eines Aerosols kombinierbar ist.

7. Steuerkörper, der mit einer Kartusche (104) koppelbar ist, die mit einem Heizelement (220) und einer Authentifizierungsvorrichtung (228) ausgerüstet ist und eine Aerosolvorläuferzusammensetzung enthält, wobei der Steuerkörper (102) mit der Kartusche (104) koppelbar ist, um eine Aerosolabgabevorrichtung zu bilden, wobei der Steuerkörper (102) umfasst:
einen elektrischen Zweidrahtverbinder (246), der mit der Kartusche (104) gekoppelt werden kann und ausgestaltet ist, um darüber Authentifizierungssignale und Leistung zu tragen;
eine Steuerkomponente (208), an die der elektrische Zweidrahtverbinder (246) gekoppelt ist, wobei die Steuerkomponente (208) ausgestaltet ist, um die Authentifizierungssignale auszutauschen und die Leistung über den elektrischen Zweidrahtverbinder (246) zu lenken, wobei die Steuerkomponente (208) die Authentifizierungssignale von dem Zweidrahtverbinder (246) verarbeitet, um die Kartusche (104) zu authentifizieren und nach der Authentifizierung die Leistung zu dem elektrischen Zweidrahtverbinder (246) zu lenken; und
eine erste Schaltschaltung (250), die mit der Steuerkomponente (208) gekoppelt ist und Teil von Schaltschaltkreis (en) bildet, der/die zum Management der Authentifizierungssignale und der Leistung über den elektrischen Zweidrahtverbinder (246) ausgestaltet ist/sind.

8. Steuerkörper nach Anspruch 7, wobei die Authentifizierungssignale über den elektrischen Zweidrahtverbinder (246) einen Spannungspegel auf oder unter einer vorbestimmten Schwellenwertspannung haben, und die Leistung über den elektrischen Zweidrahtverbinder (246) einen Spannungspegel über der vorbestimmten Schwellenwertspannung hat.

9. Steuerkörper nach Anspruch 8, wobei die vorbestimmte Schwellenwertspannung 2,5 Volt beträgt.

10. Steuerkörper nach Anspruch 7 oder 8, wobei der/die Schaltschaltkreis(e), der/die ausgestaltet ist/ sind zum Management der Authentifizierungssignale und der Leistung über den elektrischen Zweidrahtverbinder (246), einschließt/einschließen, dass er/sie ausgestaltet ist/sind zum Empfangen und Weiterleiten eines Signals über den elektrischen Zweidrahtverbinder (246) als eines der Authentifizierungssignale in einem Moment, in dem das Signal einen Spannungspegel auf oder unter der vorbestimmten Schwellenwertspannung hat.

11. Steuerkörper nach Anspruch 10, wobei der/die Schaltschaltkreis(e), der/die ausgestaltet ist/ sind zum Empfangen und Weiterleiten des Signals, einschließt/einschließen, dass er/sie ausgestaltet ist/sind zum Empfangen einer Vielzahl von Signalen und zum Weiterleiten von Signalen der Vielzahl von Signalen über den elektrischen Zweidrahtverbinder (246) als Authentifizierungssignale, bis ein Signal von der Vielzahl der Signale einen Spannungspegel über der vorbestimmten Schwellenwertspannung hat.

12. Steuerkörper nach einem der Ansprüche 8 bis 11, wobei die Schaltschaltkreis(e), der/die ausgestaltet ist/sind zum Management der Authentifizierungssignale und der Leistung über den elektrischen Zweidrahtverbinder (246), einschließt/einschließen, dass er/sie ausgestaltet ist/sind zum Empfangen und Weiterleiten eines Signals an das Heizelement (220) als Leistung, die in einem Moment, in dem das Signal einen Spannungspegel über der vorbestimmten Schwellenwertspannung hat, dorthin gelenkt wird.

13. Steuerkörper nach einem der Ansprüche 7 bis 12, wobei die Authentifizierungssignale und die Leistung als Pulsbreitenmodulations, PWM, Signale mit jeweils einer ersten Frequenz und einer zweiten Frequenz formatiert sind, wobei die erste Frequenz mindestens zwei Mal größer als die zweite Frequenz ist.

14. Steuerkörper nach Anspruch 13, wobei der/die Schaltschaltkreis(e), der/die ausgestaltet ist/ sind zum Management der Authentifizierungssignale und der Leistung über den elektrischen Zweidrahtverbinder (246), einschließt/einschließen, dass der/die Schaltschaltkreis(e) ausgestaltet ist/sind zum Schalten eines PWM-Signals mit der ersten Frequenz über den elektrischen Zweidrahtverbinder (246) zwischen Pulsen des PWM-Signals mit der zweiten Frequenz.

15. Steuerkörper nach einem der Ansprüche 7 bis 14, wobei die Steuerkomponente (208), die ausgestaltet ist, um Leistung zu dem Heizelement zu lenken, einschließt, dass sie ausgestaltet ist, um in Reaktion auf eine Luftströmung durch mindestens einen Anteil der Aerosolabgabevorrichtung Leistung zu dem Heizelement (220) zu lenken, wobei die Luft mit Dampf, der durch Verdampfung von Komponenten der Aerosolvorläuferzusammensetzung gebildet wird, unter Bildung eines Aerosols kombinierbar ist.

## Revendications

1. Dispositif de distribution d'aérosol comprenant :
une cartouche (104) qui est équipée d'un élément chauffant (220) et d'un dispositif d'authentification (228), et contient une composition de précurseur d'aérosol ; et
un organe de commande (102) couplé à la cartouche (104) et conçu pour échanger des signaux d'authentification avec le dispositif d'authentification (228) pour authentifier la cartouche (104) pour une utilisation avec l'organe de commande (102), et uniquement dans les cas où la cartouche (104) est authentifiée, diriger une énergie vers l'élément chauffant (220) pour activer et vaporiser des composants de la composition de précurseur d'aérosol,
dans lequel l'organe de commande (102) et la cartouche (104) comprennent respectivement un connecteur électrique à deux fils (246) et un connecteur électrique à deux fils (248) correspondant couplés l'un à l'autre, et à travers lesquels les signaux d'authentification sont échangés et l'énergie est dirigée, et
dans lequel l'organe de commande (102) et la cartouche (104) comprennent en outre respectivement un premier circuit de commutation (250) et un second circuit de commutation (252), le premier circuit de commutation (250) étant couplé avec le second circuit de commutation (252) pour former un montage de circuits de commutation conçu pour gérer les signaux d'authentification et l'énergie à travers le connecteur électrique à deux fils (246).

2. Dispositif de distribution d'aérosol selon la revendication 1, dans lequel les signaux d'authentification à travers le connecteur électrique à deux fils (246) ont un niveau de tension égal ou inférieur à une tension de seuil prédéterminée, et l'énergie à travers le connecteur électrique à deux fils (246) a un niveau de tension supérieur à la tension de seuil prédéterminée, en particulier,
dans lequel la tension de seuil prédéterminée est de 2,5 volts.

3. Dispositif de distribution d'aérosol selon la revendication 2, dans lequel le montage de circuits de commutation étant configuré pour gérer les signaux d'authentification et l'énergie à travers le connecteur électrique à deux fils (246) comprend le fait d'être configuré pour recevoir et transmettre un signal au dispositif d'authentification (228) en tant que l'un des signaux d'authentification dans un cas où le signal a un niveau de tension égal ou inférieur à la tension de seuil prédéterminée, en particulier,
dans lequel le montage de circuits de commutation étant configuré pour recevoir et transmettre le signal comprend le fait d'être configuré pour recevoir une pluralité de signaux et transmettre des signaux de la pluralité de signaux au dispositif d'authentification (228) en tant que signaux d'authentification jusqu'à ce qu'un signal de la pluralité de signaux ait un niveau de tension supérieur à la tension de seuil prédéterminée.

4. Dispositif de distribution d'aérosol selon la revendication 2 ou 3, dans lequel le montage de circuits de commutation étant configuré pour gérer les signaux d'authentification et l'énergie à travers le connecteur électrique à deux fils (246) comprend le fait d'être configuré pour recevoir et transmettre un signal à l'élément chauffant (220) en tant qu'énergie dirigée vers celui-ci dans un cas dans lequel le signal a un niveau de tension supérieur à la tension de seuil prédéterminée.

5. Dispositif de distribution d'aérosol selon l'une quelconque des revendications 1 à 4, dans lequel les signaux d'authentification et l'énergie sont formatés en tant que signaux de modulation d'impulsions en durée, MID, ayant respectivement une première fréquence et une seconde fréquence, la première fréquence étant au moins deux fois plus grande que la seconde fréquence, en particulier,
dans lequel le montage de circuits de commutation étant configuré pour gérer les signaux d'authentification et l'énergie à travers le connecteur électrique à deux fils (246) comprend le montage de circuits de commutation étant configuré pour commuter un signal MID ayant la première fréquence à travers le connecteur électrique à deux fils (246) entre des impulsions d'un signal MID ayant la seconde fréquence.

6. Dispositif de distribution d'aérosol selon l'une quelconque des revendications 1 à 5, dans lequel le composant de commande (208) étant configuré pour diriger l'énergie vers l'élément chauffant (220) comprend le fait d'être configuré pour diriger l'énergie vers l'élément chauffant (220) en réponse à un écoulement d'air à travers au moins une partie du dispositif de distribution d'aérosol, l'air pouvant être combiné avec la vapeur formée par la vaporisation de composants de la composition de précurseur d'aérosol pour former un aérosol.

7. Organe de commande pouvant être couplé à une cartouche (104) qui est équipée d'un élément chauffant (220) et d'un dispositif d'authentification (228), et qui contient une composition de précurseur d'aérosol, l'organe de commande (102) pouvant être couplé avec la cartouche (104) pour former un dispositif de distribution d'aérosol, l'organe de commande (102) comprenant :
un connecteur électrique à deux fils (246) pouvant être couplé avec la cartouche (104) et configuré pour transporter des signaux d'authentification et une énergie à travers celle-ci ;
un composant de commande (208) auquel est couplé le connecteur électrique à deux fils (246), le composant de commande (208) étant configuré pour échanger les signaux d'authentification et pour diriger l'énergie à travers le connecteur électrique à deux fils (246), le composant de commande (208) traitant les signaux d'authentification provenant du connecteur à deux fils (246) pour authentifier la cartouche (104) et diriger l'énergie vers le connecteur électrique à deux fils (246) lors de l'authentification ; et
un premier circuit de commutation (250) couplé au composant de commande (208) et faisant partie d'un montage de circuits de commutation configuré pour gérer les signaux d'authentification et l'énergie à travers le connecteur électrique à deux fils (246).

8. Organe de commande selon la revendication 7, dans lequel les signaux d'authentification à travers le connecteur électrique à deux fils (246) ont un niveau de tension égal ou inférieur à une tension de seuil prédéterminée, et l'énergie à travers le connecteur électrique à deux fils (246) a un niveau de tension supérieur à la tension de seuil prédéterminée.

9. Organe de commande selon la revendication 8, dans lequel la tension de seuil prédéterminée est de 2,5 volts.

10. Organe de commande selon la revendication 7 ou 8, dans lequel le montage de circuits de commutation étant configuré pour gérer les signaux d'authentification et l'énergie à travers le connecteur électrique à deux fils (246) comprend le fait d'être configuré pour recevoir et transmettre un signal à travers le connecteur électrique à deux fils (246) en tant que l'un des signaux d'authentification dans un cas dans lequel le signal a un niveau de tension égal ou inférieur à la tension de seuil prédéterminée.

11. Organe de commande selon la revendication 10, dans lequel le montage de circuits de commutation configuré pour recevoir et transmettre le signal comprend le fait d'être configuré pour recevoir une pluralité de signaux et transmettre des signaux de la pluralité de signaux à travers le connecteur électrique à deux fils (246) en tant que signaux d'authentification jusqu'à ce qu'un signal de la pluralité de signaux ait un niveau de tension supérieur à la tension de seuil prédéterminée.

12. Organe de commande selon l'une quelconque des revendications 8 à 11, dans lequel le montage de circuits de commutation étant configuré pour gérer les signaux d'authentification et l'énergie à travers le connecteur électrique à deux fils (246) comprend le fait d'être configuré pour recevoir et transmettre un signal à l'élément chauffant (220) en tant qu'énergie dirigée vers celui-ci dans un cas dans lequel le signal a un niveau de tension supérieur à la tension de seuil prédéterminée.

13. Organe de commande selon l'une quelconque des revendications 7 à 12, dans lequel les signaux d'authentification et l'énergie sont formatés en tant que signaux de modulation d'impulsions en durée, MID, ayant respectivement une première fréquence et une seconde fréquence, la première fréquence étant au moins deux fois plus grande que la seconde fréquence.

14. Organe de commande selon la revendication 13, dans lequel le montage de circuits de commutation étant configuré pour gérer les signaux d'authentification et l'énergie à travers le connecteur électrique à deux fils (246) comprend le fait que le montage de circuits de commutation est configuré pour commuter un signal MID ayant la première fréquence à travers le connecteur électrique à deux fils (246) entre des impulsions d'un signal MID ayant la seconde fréquence.

15. Organe de commande selon l'une quelconque des revendications 7 à 14, dans lequel le composant de commande (208) étant configuré pour diriger l'énergie vers l'élément chauffant comprend le fait d'être configuré pour diriger l'énergie vers l'élément chauffant (220) en réponse à un écoulement d'air à travers au moins une partie du dispositif de distribution d'aérosol, l'air pouvant être combiné avec la vapeur formée par la vaporisation de composants de la composition de précurseur d'aérosol pour former un aérosol.
